# EUROPEAN PATENT SPECIFICATION

(11) **EP 3 542 772 B1**
(45) Date of publication and mention of the grant of the patent: **16.08.2023**
(21) Application number: 17871780.7
(22) Date of filing: 29.09.2017
(51) Int. Cl.: A61J 1/20, A61M 5/00, A61M 5/14, A61M 5/142

(54) **DRUG SOLUTION FILLING UNIT, DRUG SOLUTION FILLING SET, AND FILLING ADAPTER**
WIRKSTOFFLÖSUNGBEFÜLLUNGSEINHEIT, WIRKSTOFFLÖSUNGBEFÜLLUNGSSET UND FÜLLADAPTER
UNITÉ DE REMPLISSAGE DE SOLUTION MÉDICAMENTEUSE, ENSEMBLE DE REMPLISSAGE DE SOLUTION MÉDICAMENTEUSE ET ADAPTATEUR DE REMPLISSAGE

(30) Priority: 21.11.2016 JP 2016226230
(43) Date of publication of application: 25.09.2019
(73) Proprietor: Terumo Kabushiki Kaisha, Tokyo 151-0072 (JP)
(72) Inventor: UCHIYAMA, Joji, Ashigarakami-gun Kanagawa 259-0151 (JP); NAKAMOTO, Masato, Ashigarakami-gun Kanagawa 259-0151 (JP); YAMAZAKI, Tsuyoshi, Ichinoseki-shi Iwate 029-0803 (JP)
(74) Representative: Casalonga
(86) International application number: PCT/JP2017/035682
(87) International publication number: WO 2018/092437

(56) References cited:
- WO-A1-2011/030787
- WO-A1-2015/115435
- WO-A1-2016/051999
- WO-A1-2016/141082
- JP-A- H 119 656
- JP-A- H08 257 101
- JP-A- 2003 126 221
- JP-A- 2009 153 720
- JP-A- 2016 064 015
- US-A1- 2012 186 698
- US-A1- 2015 209 232

## Description

### Technical Field

The present invention relates to a drug solution filling unit, a drug solution filling set, and a filling adapter for filling a drug solution into a drug solution reservoir instrument used in a drug solution administration device. In particular the invention relates to a drug solution filling unit according to the preamble of claim 1, such as it is e.g. known from JP 2016 064 015 A and US2012/186698 A1.

### Background Art

A portable drug solution administration device (a so-called insulin pump) that can be carried in a state of being attached to a patient' s body, clothes, or the like is known as a medical device used for administration of a drug solution (for example, insulin) to a diabetes patient or the like.

In related art, as a portable drug solution administration device, there is such a device as described in Patent Literature 1. The drug solution administration device described in Patent Literature 1 includes a drug solution reservoir portion that stores a drug solution, and a liquid sending mechanism such as a plunger that sends the drug solution from the drug solution reservoir portion.

### Citation List

### Patent Literature

Patent Literature 1: JP 2010-501283 A

### Summary of Invention

### Technical Problem

In general, when the drug solution administration device reaches the user's hand, the drug solution reservoir portion is not filled with the drug solution. Therefore, when the drug solution is administered, the user performs an operation of connecting a drug solution container (for example, a vial bottle) containing the drug solution with the drug solution reservoir portion of the drug solution administration device, and delivering the drug solution from the drug solution container to the drug solution reservoir portion.

Further, when the operation of delivering the drug solution from the drug solution container to the drug solution reservoir portion is performed as described above, the drug solution container and the drug solution reservoir portion are in fluid communication via a solution delivering flow path through which the drug solution can flow. For example, a needle tube that can penetrate a seal member for sealing the drug solution container and a seal member for sealing the drug solution reservoir portion can be used as the solution delivering flow path.

However, if a state in which the needle tube is made to penetrate through the seal member of the drug solution reservoir portion is maintained over a relatively long period of time, the sealing property (airtightness) of a portion (a needle defect periphery) of the seal member through which the needle tube penetrates decreases. For this reason, it is desirable that the operation of causing the needle tube to penetrate through the seal member of the drug solution reservoir portion be performed immediately before the operation of delivering the drug solution to the drug solution reservoir portion.

Further, if the drug solution reservoir portion and the needle tube are provided to the user in an individually separated state, since the user is forced to handle the needle tube with user's fingers, the operation of causing the needle tube to penetrate the seal member of the drug solution reservoir portion is very troublesome.

The present invention has been made in view of the above-described problems, and an object of the present invention is to provide a drug solution filling unit, a drug solution filling set, and a filling adapter capable of preventing a decrease in the sealing property of a seal member of a drug solution reservoir portion and easily performing an operation of causing a needle tube to penetrate the seal member of the drug solution reservoir portion.

### Solution to Problem

### Advantageous Effects of Invention

According to the present invention, a user can prevent the first needle tube of the filling adapter from penetrating the first seal member of the drug solution reservoir portion before the filling of the drug solution to the drug solution reservoir instrument is started, in a state in which the filling adapter has been mounted to the drug solution reservoir instrument. Further, the user can make the first needle tube of the filling adapter penetrate the first seal member by a simple operation of changing the relative position of the filling adapter with respect to the drug solution reservoir instrument. Therefore, the present invention can prevent the sealing property of the first seal member of the drug solution reservoir portion from being lowered, and can easily perform the operation of causing the first needle tube to penetrate the first seal member of the drug solution reservoir portion. The invention is defined by the subject-matter of independent claim 1. Dependent claims 2 and 3 provide further advantageous embodiments.

### Brief Description of Drawings

Fig. 1 is a perspective view illustrating a drug solution filling set according to an embodiment.
Fig. 2 schematically illustrates an overall configuration of the drug solution filling set.
Fig. 3 is a front view illustrating the drug solution reservoir instrument.
Fig. 4 is a front view illustrating a filling adapter.
Fig. 5 is a cross-sectional view of the filling adapter.
Fig. 6 is a perspective view illustrating the filling adapter.
Fig. 7 is a perspective view illustrating the filling adapter.
Fig. 8 is a perspective view illustrating a state when the filling adapter is mounted to a drug solution reservoir instrument.
Fig. 9 is a perspective view illustrating a state in which the filling adapter has been mounted to the drug solution reservoir instrument.
Fig. 10 is a front view illustrating a drug solution filling device.
Fig. 11 is a perspective view illustrating a state in which the drug solution filling unit has been mounted to the drug solution filling device.
Fig. 12 is an enlarged view illustrating a part of the drug solution filling device.
Fig. 13 is an enlarged view illustrating a part of the drug solution filling device.
Fig. 14 is a diagram for describing a holding mechanism included in the drug solution filling device.
Fig. 15 is a diagram schematically illustrating an operation of mounting the drug solution filling unit to the drug solution filling device.
Figs. 16(A) to 16(C) are diagrams schematically illustrating a procedure of a filling operation using the drug solution filling device.
Fig. 17 is a perspective view illustrating a state when the drug solution filling unit is mounted to the drug solution filling device.
Fig. 18 is a cross-sectional view illustrating a state when the drug solution filling unit is mounted to the drug solution filling device, and is a cross-sectional view along an arrow 18A-18A of Fig. 17.
Fig. 19 is a perspective view illustrating a state when the drug solution filling unit is mounted to the drug solution filling device.
Fig. 20 is a perspective view illustrating a state when the drug solution filling unit is mounted to the drug solution filling device.
Fig. 21 is a cross-sectional view illustrating a state when the drug solution filling unit is mounted to the drug solution filling device, corresponding to Fig. 18.
Fig. 22 is a cross-sectional view illustrating a state when the drug solution filling unit is mounted to the drug solution filling device, corresponding to Fig. 18.
Fig. 23 is a cross-sectional view illustrating a state when a drug solution filling unit is mounted to the drug solution filling device, corresponding to Fig. 18.
Fig. 24 is a cross-sectional view illustrating a state when the drug solution filling unit is mounted to the drug solution filling device.
Fig. 25 is an exploded perspective view of a drug solution administration device according to the embodiment.

Hereinafter, embodiments of the present invention will be described with reference to the accompanying drawings. It should be noted that the following description does not limit the technical scope and the meaning of the terms described in the claims. Also, dimensional ratios of the drawings are exaggerated for convenience of explanation and may differ from the actual ratios.

In the description of this embodiment, an example in which the present invention is applied to an insulin pump, which is a portable drug solution administration device, will be described. However, the present invention can be widely applied to various drug solution administration devices used for the purpose of administration of the drug solution.

Hereinafter, a drug solution filling set 10, a drug solution filling unit 100, a drug solution reservoir instrument 200, a filling adapter 300, a drug solution filling device 400, a drug solution container 500, and a drug solution administration device 600 will be described.

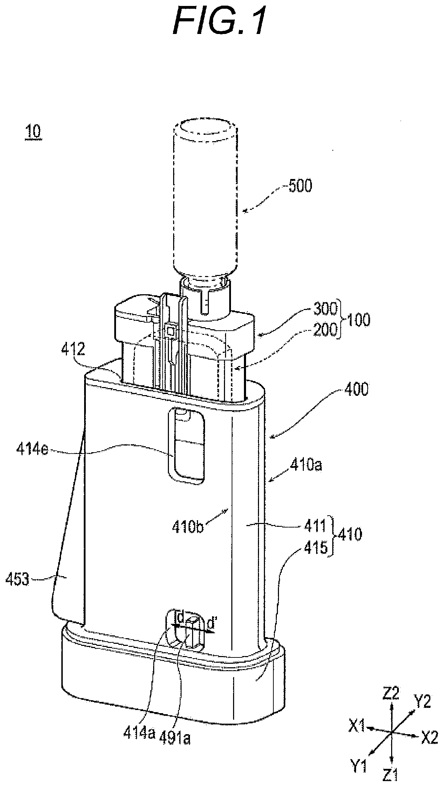

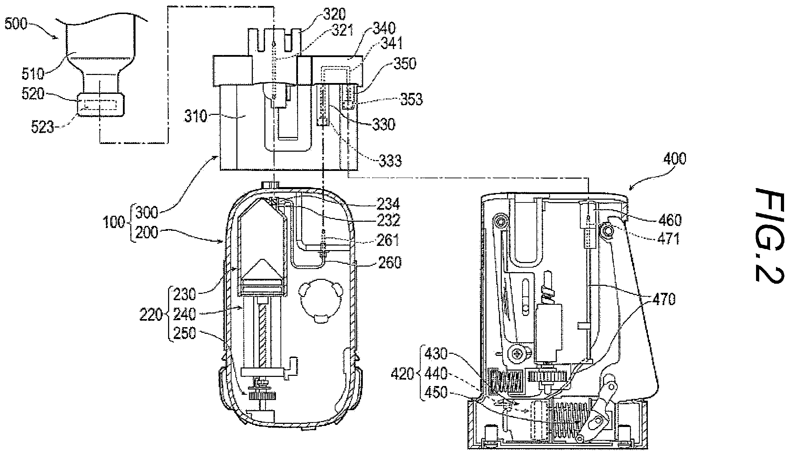

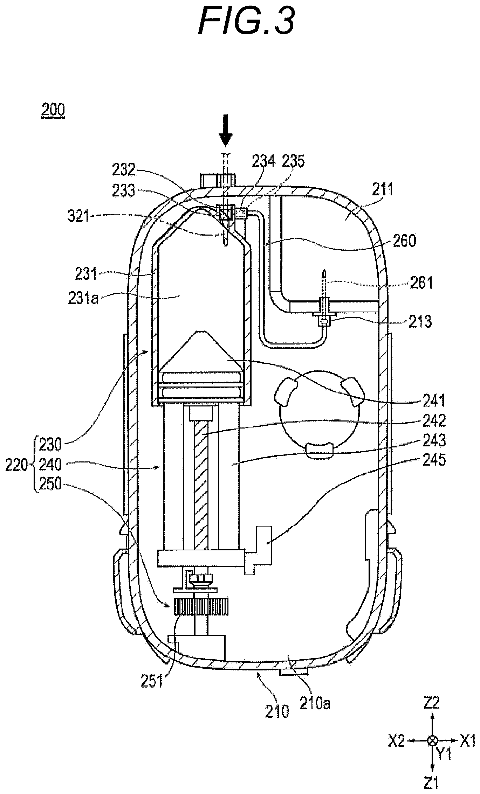

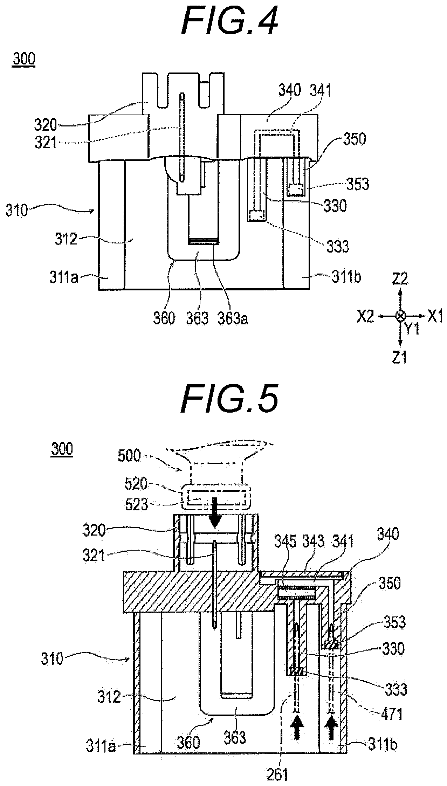

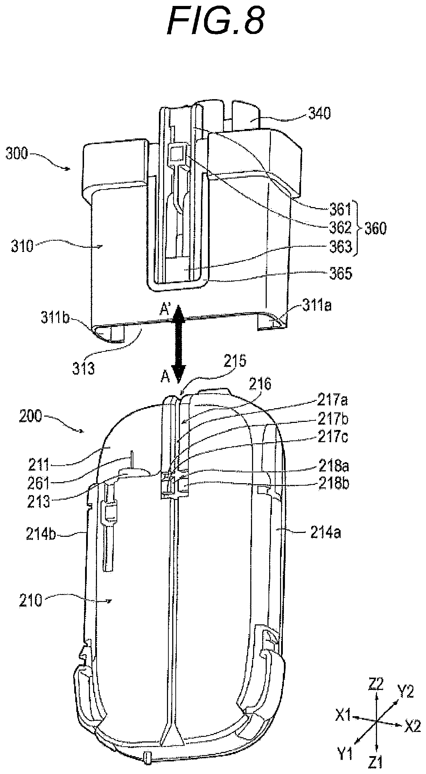

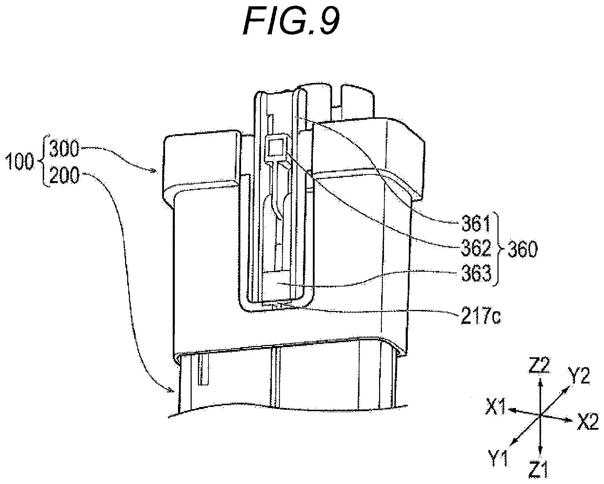

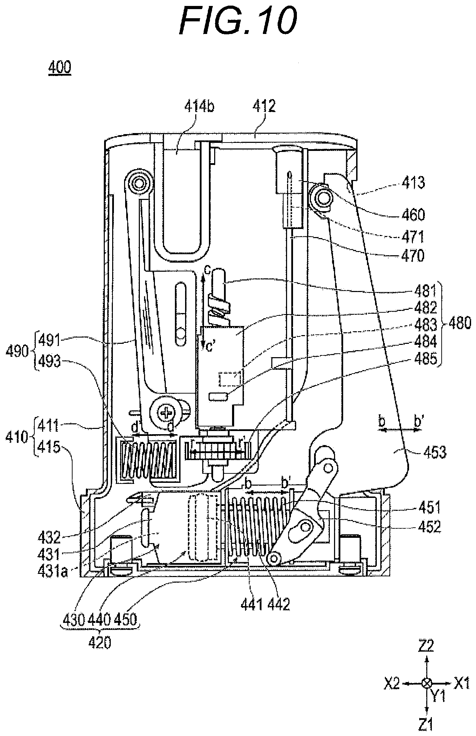

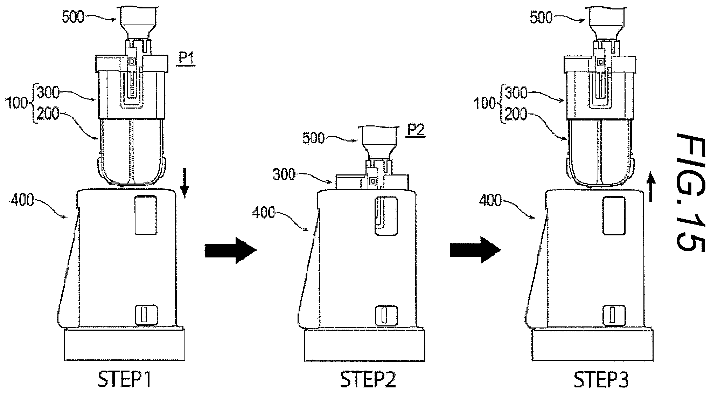

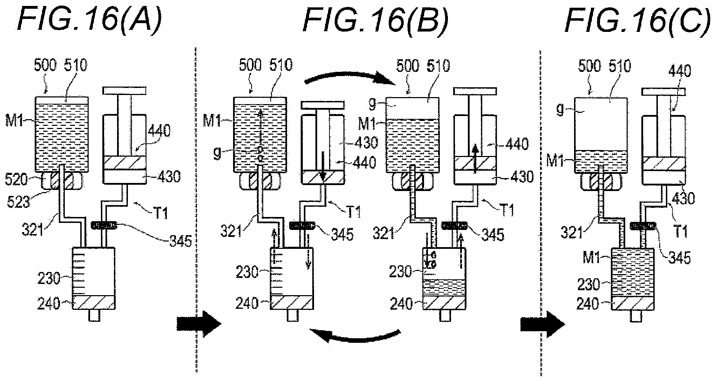

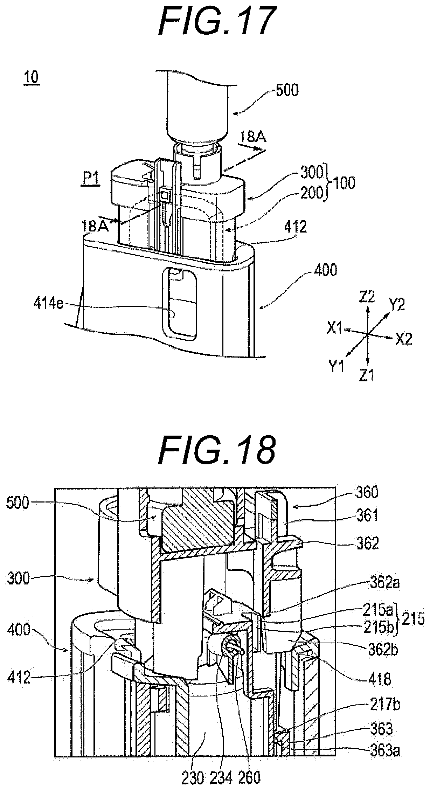

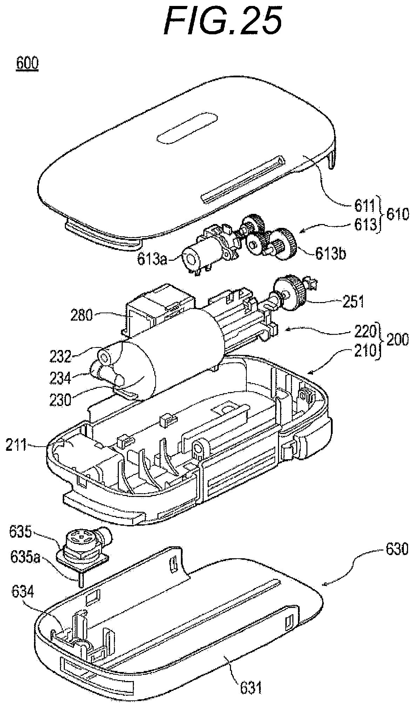

Figs. 1 and 2 are diagrams illustrating the drug solution filling set 10, Fig. 3 is a diagram illustrating the drug solution reservoir instrument 200, Figs. 4 to 7 are diagrams illustrating the filling adapter 300, Figs. 8 and 9 are diagrams illustrating the drug solution filling unit 100, Figs. 10 to 14 are diagrams illustrating the drug solution filling device 400, Figs. 15 and 16 are diagrams illustrating usage examples of the drug solution filling set 10, Figs. 17 to 24 are diagrams illustrating an operation of a lock mechanism 360, and Fig. 25 is a diagram illustrating the drug solution administration device 600.

Further, in each drawing, a width direction of the drug solution filling set 10 (the drug solution reservoir instrument 200, the filling adapter 300, and the drug solution filling device 400) is indicated by X1 axis-X2 axis, a depth direction of the drug solution filling set 10 is indicated by Y1 axis-Y2 axis, and a height direction of the drug solution filling set 10 is indicated by Z1 axis-Z2 axis.

### <Drug solution filling set>

Fig. 1 illustrates a drug solution filling set 10 according to this embodiment.

The drug solution filling set 10 has the drug solution filling unit 100, and the drug solution filling device 400 used when filling the drug solution reservoir instrument 200 with a drug solution. The drug solution filling unit 100 has the drug solution reservoir instrument 200 which stores the drug solution to be administered to a patient, and the filling adapter 300 detachably mounted to the drug solution reservoir instrument 200.

As illustrated in Fig. 25, the drug solution reservoir instrument 200 is used as a component constituting a part (a disposable portion) of the drug solution administration device 600. As illustrated in Fig. 1, the filling adapter 300 is instrument for connecting the drug solution container 500 (for example, a vial bottle) containing the drug solution and the drug solution reservoir instrument 200. The drug solution filling device 400 is instrument for delivering the drug solution from the drug solution container 500 to the drug solution reservoir instrument 200.

A flow path for connecting the drug solution container 500 and each part of the drug solution filling set 10 will be described with reference to Fig. 2.

Prior to use of the drug solution administration device 600, a user (for example, a patient or a medical person such as a doctor who administers the drug solution) who uses the drug solution administration device 600 performs an operation of filling the drug solution reservoir portion 230 of the drug solution reservoir instrument 200 with the drug solution. In this operation, the user connects the drug solution container 500 and the drug solution reservoir instrument 200 via the filling adapter 300. Further, the user connects the drug solution reservoir instrument 200 and the drug solution filling device 400 via the filling adapter 300. When each member is connected as described above, a flow path through which the drug solution container 500, the filling adapter 300, the drug solution reservoir instrument 200, and the drug solution filling device 400 communicate with each other is formed.

As illustrated in Fig. 2, the aforementioned flow path is formed to allow a main body portion 510 of the drug solution container 500, a first needle tube 321 included in the filling adapter 300, the drug solution reservoir portion 230 of the drug solution reservoir instrument 200, a first connection port portion (corresponding to a "first port portion") 232 of the drug solution reservoir portion 230, a second connection port portion (corresponding to a "third port portion") 234 of the drug solution reservoir portion 230, a tube 260 of the drug solution reservoir instrument 200, a second needle tube 261 of the drug solution reservoir instrument 200, a first connection port portion (corresponding to a "second port portion") 330 of the filling adapter 300, a flow passage 341 of the filling adapter 300, a second connection port portion 350 of the filling adapter 300, a third needle tube 471 of the drug solution filling device 400, a tube 470 of the drug solution filling device 400, and a syringe 430 of the drug solution filling device 400 to communicate with each other.

The user can deliver the drug solution from the drug solution container 500 to the drug solution reservoir portion 230, by operating the drug solution filling device 400 as will be described later, in a state of connecting the drug solution container 500 and the drug solution filling set 10 to form the flow path (see Fig. 16).

### <Drug solution filling unit>

When the drug solution reservoir instrument 200 and the filling adapter 300 constituting the drug solution filling unit 100 are provided to the user, they can be accommodated in a predetermined container such as a packaging material in a state of being connected to each other.

When using the drug solution filling unit 100, the user extracts the drug solution filling unit 100 from a predetermined packaging material or the like. Next, the drug solution container 500 is connected to the filling adapter 300 of the drug solution filling unit 100 (STEP 1 of Fig. 15) . Next, the drug solution filling unit 100 is mounted to the drug solution filling device 400 to perform a filling operation (STEP 2 of Fig. 15) . Next, the drug solution filling unit 100 is detached from the drug solution filling device 400 (STEP 3 of Fig. 15) . After the user detaches the drug solution container 500 from the filling adapter 300, the user detaches the drug solution reservoir instrument 200 from the filling adapter 300. After being detached from the filling adapter 300, the drug solution reservoir instrument 200 is used as a disposer portion of the drug solution administration device 600 (see Fig. 25).

### <Drug solution reservoir instrument>

As illustrated in Fig. 3, the drug solution reservoir instrument 200 has a drug solution reservoir portion 230 for storing the drug solution, and an exterior case 210 for accommodating the drug solution reservoir portion 230.

The exterior case 210 is a casing in which an internal space 210a capable of accommodating each constituent member of the drug solution reservoir instrument 200 is formed.

The exterior case 210 is provided with a protrusion accommodating portion 211 protruding to the inside of the internal space 210a. The protrusion accommodating portion 211 is provided with a port 213 to which a tube 260 disposed inside the internal space 210a is connected. Further, the second needle tube 261 is attached to the port 213. As illustrated in Fig. 8, a part of the port 213 and a part of the second needle tube 261 are exposed to the outside of the exterior case 210 on the back side of the exterior case 210.

Further, as illustrated in Fig. 8, the exterior case 210 is provided with a first lock portion 215 and a second lock portion 216 which cooperate with a lock mechanism 360 of the filling adapter 300 to be described later to restrict movement of the filling adapter 300.

As illustrated in Fig. 18, the first lock portion 215 has an abutting portion 215a on which a stopper portion 362 of the lock mechanism 360 of the filling adapter 300 abuts in a state in which the filling adapter 300 has been mounted to the drug solution reservoir instrument 200, and a groove portion 215b extending downward along the height direction (a Z1-Z2 direction of Fig. 3) of the exterior case 210 from the abutting portion 215a.

As illustrated in Fig. 8, the second lock portion 216 has a first groove portion 217a extending along the height direction of the exterior case 210, a second groove portion 217b (corresponding to a "groove portion") extending in a direction (a X1-X2 direction in the drawing) intersecting with the first groove portion 217a, and a third groove portion (corresponding to a "groove portion") 217c extending substantially in parallel with the second groove portion 217b.

The second groove portion 217b is formed on the upper side (an upper side of Fig. 8) of the third groove portion 217c in the height direction of the exterior case 210. Further, between the second groove portion 217b and the third groove portion 217c in the height direction of the exterior case 210, a pair of convex portions 218a and 218b disposed to face each other along the width direction (the X1-X2 direction in the drawing) of the exterior case 210 is formed. An inclined wall in which a thickness gradually decreases toward the upper portion side of the exterior case 210 is formed at the upper end portions of the respective convex portions 218a and 218b.

As illustrated in Fig. 3, the drug solution reservoir instrument 200 has a liquid sending mechanism 220 for sending the drug solution to a patient. The liquid sending mechanism 220 has a drug solution reservoir portion 230, a pusher member 240, and a drive portion 250.

The drug solution reservoir portion 230 has a reservoir portion main body 231 in which an internal space 231a capable of containing the drug solution is formed, a first connection port portion 232 provided on one end portion side (the upper side of Fig. 3) of the reservoir portion main body 231, and a second connection port portion 234 provided at a position different from the first connection port portion 232 in the reservoir portion main body 231.

The reservoir portion main body 231 has a conical shape in which one end portion side tapers. The other end portion side (the lower side of Fig. 3) of the reservoir portion main body 231 is an open end opened to the outside. A gasket 241 of the pusher member 240 is inserted into the internal space 231a of the reservoir portion main body 231.

The first connection port portion 232 opens upward in Fig. 3. A seal member (corresponding to a "first seal member") 233 is disposed in the first connection port portion 232. The first needle tube 321 of the filling adapter 300 is pierced into the seal member 233.

The second connection port portion 234 opens toward a right direction in Fig. 3. A seal member 235 is disposed in the second connection port portion 234. The tube 260 of the drug solution reservoir portion 230 is connected to the seal member 235. A second needle tube 261 is attached to one end portion of the tube 260 of the drug solution reservoir portion 230. The second needle tube 261 is pierced into a seal member (corresponding to a "second seal member") 333 disposed in the first connection port portion 330 of the filling adapter 300 (see Fig. 2).

The drug solution reservoir instrument 200 and the filling adapter 300 are in a state of being connected to each other when provided to a user as the drug solution filling unit 100. However, the first needle tube 321 of the filling adapter 300 is held at a position at which it does not penetrate the seal member 233 disposed in the first connection port portion 232 of the drug solution reservoir instrument 200. Similarly, when the drug solution filling unit 100 is provided to the user, the second needle tube 261 of the drug solution reservoir instrument 200 is held at a position at which it does not penetrate the seal member 333 disposed at the first connection port portion 330 of the filling adapter 300. As will be described later, when filling the drug solution reservoir instrument 200 with the drug solution, the user operates the drug solution reservoir instrument 200 and the filling adapter 300, thereby causing the first needle tube 321 of the filling adapter 300 to penetrate the seal member 233 of the first connection port portion 232 of the drug solution reservoir instrument 200, and causing the second needle tube 261 of the drug solution reservoir instrument 200 to penetrate the seal member 333 of the first connection port portion 330 of the filling adapter 300.

As illustrated in Fig. 3, the pusher member 240 of the liquid sending mechanism 220 includes a gasket 241 accommodated in the reservoir portion main body 231, a feed screw portion 242 for moving the gasket 241, a shaft portion 243 attached to the feed screw portion 242, and an engaging piece 245 attached to the shaft portion 243.

The feed screw portion 242 rotates as the rotational power is transmitted from the drive portion 250. When the feed screw portion 242 rotates, the shaft portion 243 moves along the extending direction (the vertical direction of Fig. 3) of the reservoir portion main body 231 in conjunction with the rotation thereof . The gasket 241 moves inside the reservoir portion main body 231 in conjunction with the movement of the shaft portion 243. When the gasket 241 moves forward (moves upward of Fig. 3) while using the drug solution administration device 600 (see Fig. 25), the drug solution is sent out from the drug solution reservoir portion 230 via the second connection port portion 234.

The engaging piece 245 provided on the shaft portion 243 is configured to be connectable with the engaging portion 483 (see Fig. 10) provided in the drug solution filling device 400. As will be described later, by adjusting the position of the engaging piece 245 in a state in which the drug solution filling unit 100 has been mounted to the drug solution filling device 400, it is possible to adjust an amount (an initial filling amount) of the drug solution to be filled in the drug solution reservoir portion 230.

The drive portion 250 of the liquid sending mechanism 220 is a mechanism for transmitting the rotational power to the feed screw portion 242. The drive portion 250 has a gear group 251 that freely meshes with a gear group 613b (see Fig. 25) of a reuse portion 610 of the drug solution administration device 600. When the drug solution reservoir instrument 200 is used as the disposer portion of the drug solution administration device 600 to administer the drug solution to a patient, the rotational power is transmitted from the gear group 613b of the reuse portion 610 to rotate the gear group 251. When the gear group 251 rotates, the feed screw portion 242 rotates in conjunction with the rotation of the gear group 251.

### <Filling adapter>

As illustrated in Figs. 4 and 5, the filling adapter 300 includes an adapter main body portion 310 to which the first needle tube 321 is attached, and a lock mechanism 360 provided integrally with the adapter main body portion 310.

The adapter main body portion 310 has a connection portion 320 to which the drug solution container 500 is connected, a first connection port portion (corresponding to a "second port portion") 330 through which the drug solution can flow, a flow path partition portion 340 in which a flow passage 341 is partitioned through which a gas g such as air can flow, and a second connection port portion 350 through which the drug solution can flow.

A seal member 333 is disposed in the first connection port portion 330 of the adapter main body portion 310. As described above, the second needle tube 261 of the drug solution reservoir instrument 200 is pierced to the seal member 333, but at the stage in which the drug solution filling unit 100 is provided to the user, the second needle tube 261 is held at a position where it does not penetrate through the seal member 333.

A seal member 353 is disposed in the second connection port portion 350 of the adapter main body portion 310. As illustrated in Fig. 5, the third needle tube 471 of the drug solution filling device 400 penetrates through the seal member 353.

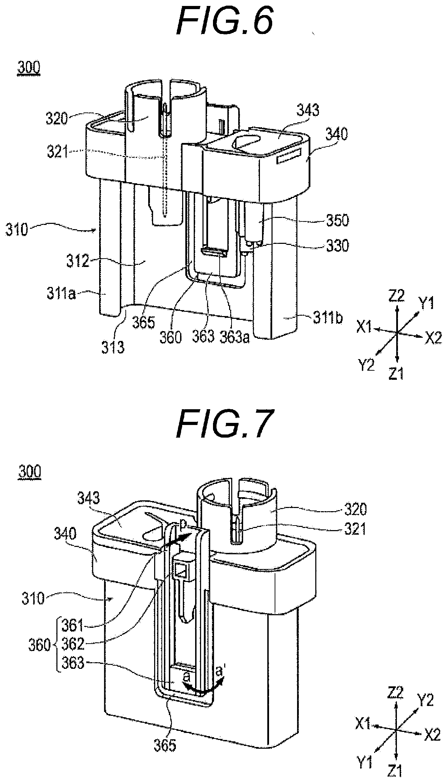

As illustrated in Fig. 6, the adapter main body portion 310 has a pair of slide guide portions 311a and 311b formed on both side surfaces of the adapter main body portion 310, an inner surface guide portion 312 formed on the inner surface of the adapter main body portion 310, and an insertion port 313 formed at the lower end portion of the adapter main body portion 310.

As illustrated in Fig. 8, the slide guide portions 311a and 311b of the adapter main body portion 310 are disposed to be in contact with the respective side surfaces 214a and 214b of the exterior case 210 of the drug solution reservoir instrument 200 in a state in which the filling adapter 300 has been mounted to the drug solution reservoir instrument 200. Further, the inner surface guide portion 312 of the adapter main body portion 310 is disposed to be in contact with the back surface of the exterior case 210 of the drug solution reservoir instrument 200. The respective slide guide portions 311a and 311b and the inner surface guide portion 312 guide the sliding movement of the adapter main body portion 310 when moving the adapter main body portion 310 with respect to the exterior case 210.

The connection portion 320 of the adapter main body portion 310 is configured so that a lid portion 520 of the drug solution container 500 can be inserted. One end portion side of the first needle tube 321 is disposed to protrude from the connection portion 320 so as to face the outside. When the lid portion 520 of the drug solution container 500 is inserted into the connection portion 320, the lid portion 520 is provisionally fixed to the connection portion 320. Further, when the lid portion 520 is connected to the connection portion 320, the first needle tube 321 penetrates the seal member 523 disposed in the lid portion 520 of the drug solution container 500. The main body portion 510 of the drug solution container 500 communicates with the drug solution reservoir portion 230 via the first needle tube 321 (see Fig. 2).

As illustrated in Fig. 5, the first connection port portion 330 and the second connection port portion 350 provided in the adapter main body portion 310 communicate with each other via the flow passage 341 partitioned inside the adapter main body portion 310. The flow passage 341 is formed by covering a space formed inside the adapter main body portion 310 with a lid member 343 disposed on the upper portion side of the adapter main body portion 310. The lid member 343 is fixed to the adapter main body portion 310 by welding or bonding.

A ventilation filter 345 is disposed in the flow passage 341 of the adapter main body portion 310. The ventilation filter 345 allows passage of a gas such as air, while preventing passage of a liquid such as a drug solution. As will be described later, the ventilation filter 345 prevents the drug solution from flowing into the drug solution filling device 400 side via the flow passage 341, when storing the drug solution in the drug solution reservoir instrument 200, using the drug solution filling device 400 (see Fig. 16(C)).

As illustrated in Fig. 7, the lock mechanism 360 provided in the adapter main body portion 310 has a lever portion 361, a stopper portion 362, and a claw portion 363.

The lever portion 361 of the lock mechanism 360 is formed of a plate-shaped member recessed inside the adapter main body portion 310.

The stopper portion 362 of the lock mechanism 360 has portions 362a and 362b protruding toward the inside of the adapter main body portion 310 (see Fig. 18) . A portion 362a protruding toward the inside of the adapter main body portion 310 in the stopper portion 362 cooperates with the first lock portion 215 of the drug solution reservoir instrument 200 to restrict the movement of the filling adapter 300 as will be described later.

The claw portion 363 of the lock mechanism 360 is formed on the lower end side of the lock mechanism 360. As illustrated in Fig. 6, the claw portion 363 has a protruding portion 363a protruding to the inside of the adapter main body portion 310. The protruding portion 363a of the claw portion 363 can be caught by the respective groove portions 217b and 217c (see Fig. 8) of the second lock portion 216 of the drug solution reservoir instrument 200.

The lock mechanism 360 is formed on the back surface of the adapter main body portion 310 (a surface arranged to face the back surface side of the drug solution reservoir instrument 200). Further, a notch 365 for making the lock mechanism 360 movable is formed around the portion of the adapter main body portion 310 in which the lock mechanism 360 is formed.

The lock mechanism 360 is connected to the adapter main body portion 310 via a lever portion 361 located on the upper portion side of the lock mechanism 360. When the lever portion 361 is pressed along the depth direction of the adapter main body portion 310 as indicated by an arrow p of Fig. 7, the lock mechanism 360 moves (swings) in a direction indicated by an arrow a from the lever portion 361 as a starting point. Further, when the pressing of the lever portion 361 is released, the lock mechanism 360 is movable in a direction indicated by an arrow a' and returns to the original position before pressing.

When the drug solution filling unit 100 is provided to the user, as illustrated in Fig. 18, the end portion (the lower end portion in Fig. 8) 362a of the stopper portion 362 of the lock mechanism 360 abuts on the abutting portion 215a of the first lock portion 215. Further, when the drug solution filling unit 100 is provided to the user, the claw portion 363 of the lock mechanism 360 is caught by the second groove portion 217b of the second lock portion 216. The relative movement of the filling adapter 300 with respect to the drug solution reservoir instrument 200 is restricted by the abutment between the predetermined portion 362a of the stopper portion 362 and the abutting portion 215a, and the catching of the claw portion 363 with respect to the second groove portion 217b.

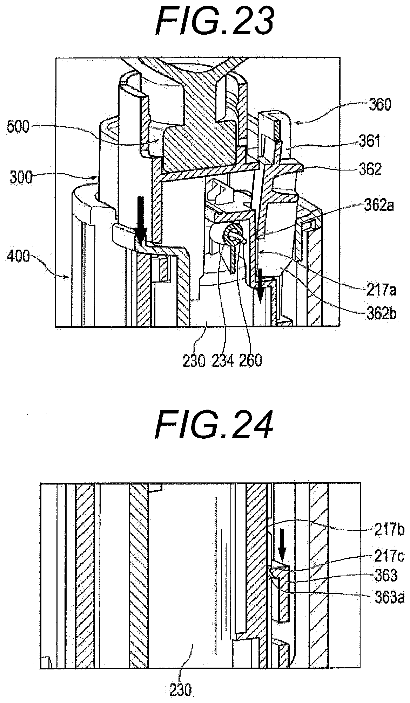

As illustrated by an arrow A in Fig. 8, the filling adapter 300 can be mounted to the drug solution reservoir instrument 200 by relatively moving the filling adapter 300 to approach the drug solution reservoir instrument 200. Further, as illustrated by an arrow A' in Fig. 8, the filling adapter 300 can be detached from the drug solution reservoir instrument 200 by relatively moving the filling adapter 300 away from the drug solution reservoir instrument 200. Further, when detaching the filling adapter 300 from the drug solution reservoir instrument 200, as will be described later, an operation of releasing the catching of the claw portion 363 with respect to the third groove portion 217c of the second lock portion 216 is performed (see Fig. 24) .

Fig. 9 illustrates a state in which the filling adapter 300 has been mounted to the drug solution reservoir instrument 200. The drug solution filling unit 100 according to this embodiment is provided to the user in a state in which the filling adapter 300 has been mounted to the drug solution reservoir instrument 200 in this way. In addition, as described above, the drug solution filling unit 100 is provided to the user in a state in which the relative movement of the filling adapter 300 with respect to the drug solution reservoir instrument 200 is restricted.

More specifically, when the drug solution filling unit 100 is provided to the user, the filling adapter 300 is held at a position (a non-penetrating position P1) at which the first needle tube 321 on the filling adapter 300 side does not penetrate the seal member 233 disposed in the drug solution reservoir portion 230, and the second needle tube 261 on the drug solution reservoir instrument 200 side does not penetrate the seal member 333 disposed in the first connection port portion 330 of the filling adapter 300. Further, as will be described later, when the user releases the restriction of the lock mechanism 360 at the time of filling the drug solution to the drug solution reservoir instrument 200, the filling adapter 300 can move to a position (a penetrating position P2) at which the first needle tube 321 on the filling adapter 300 side penetrates the seal member 233 disposed on the drug solution reservoir portion 230, and the second needle tube 261 on the drug solution reservoir instrument 200 side penetrates the seal member 333 disposed in the first connection port portion 330 of the filling adapter 300.

### <Drug solution filling device>

As illustrated in Fig. 10, the drug solution filling device 400 includes an exterior case 410 that accommodates each constituent member of the drug solution filling device 400, a filling mechanism 420 that sends the drug solution to the drug solution reservoir instrument 200, a filling device side connection port portion 460 to which the second connection port portion 350 of the filling adapter 300 is connected, a tube 470 to which a third needle tube 471 penetrating the seal member 353 disposed at the second connection port portion 350 of the filling adapter 300 is attached, a filling amount adjustment mechanism 480 which adjusts the amount of drug solution stored in the drug solution reservoir instrument 200, and a holding mechanism 490 which holds the drug solution reservoir instrument 200 at a fixed position with respect to the drug solution filling device 400.

The exterior case 410 of the drug solution filling device 400 has a main body portion 411 and a pedestal portion 415. The pedestal portion 415 supports the drug solution filling device 400 to the floor surface, the desk or the like, and makes the drug solution filling device 400 stand upright. The main body portion 411 is formed to extend upward from the pedestal portion 415, and its side surface is formed in a rounded shape. The filling amount adjustment mechanism 480 and the holding mechanism 490 are disposed in the main body portion 411, and the respective constituent members of the filling mechanism 420 are disposed in the pedestal portion 415.

An upper opening portion 412 for inserting the drug solution filling unit 100 is formed at the upper end portion of the exterior case 410 (see Fig. 1) . A side surface opening portion 413 from which the lever portion 453 of the filling mechanism 420 protrudes is formed on the side surface of the exterior case 410.

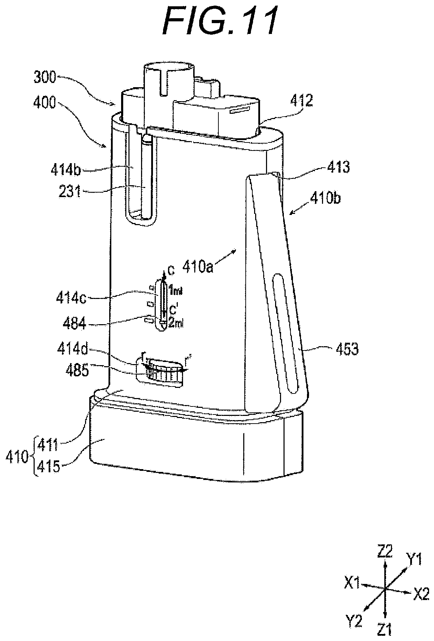

As illustrated in Fig. 11, on one surface 410a side in the front direction of the exterior case 410, in a state in which the drug solution filling unit 100 has been mounted to the drug solution filling device 400, a window portion 414b that makes the reservoir portion main body 231 visible from the outside, a window portion 414c for exposing the indication piece 484 of the filling amount adjustment mechanism 480 to the outside, and a window portion 414d for exposing the rotary dial 485 of the filling amount adjustment mechanism 480 to the outside are formed.

Further, as illustrated in Fig. 1, on the other surface 410b side in the front direction of the exterior case 410, in a state in which the drug solution filling unit 100 has been mounted to the drug solution filling device 400, a window portion 414a for exposing the lever portion 491a of the holding mechanism 490 to the outside, and a window portion 414e for making a part of the drug solution filling unit 100 visible from the outside are formed.

As illustrated in Fig. 10, the filling mechanism 420 has a syringe 430, a pusher member 440, and a drive portion 450.

The syringe 430 has a syringe main body 431 including an internal space 431a capable of storing a gas g such as air, and a port portion 432 provided in the syringe main body 431.

The port portion 432 of the syringe 430 is connected to the tube 470 accommodated in the exterior case 410. The gas g such as air sent from the syringe 430 via the port portion 432 is sent to the second connection port portion 350 of the filling adapter 300 via the tube 470 and the third needle tube 471.

The pusher member 440 has a gasket 441 accommodated in the internal space 431a of the syringe main body 431, and the shaft portion 442 attached to the gasket 441.

The shaft portion 442 is connected to the link mechanism 452 of the drive portion 450. The shaft portion 442 moves in conjunction with a manipulation of gripping the lever portion 453 and a manipulation of releasing the gripping. The gasket 441 disposed at the tip of the shaft portion 442 moves along an extending direction (a left-right direction in Fig. 10) of the internal space 431a of the syringe main body 431 with the movement of the shaft portion 442.

The drive portion 450 of the filling mechanism 420 has an urging member 451 that imparts an urging force to the shaft portion 442 of the pusher member 440, a predetermined link mechanism 452, and a lever portion 453 connected to the pusher member 440 via the link mechanism 452.

The urging member 451 can be constituted by, for example, a known coil spring. The urging member 451 applies the urging force (an elastic force) in a direction of an arrow b' in Fig. 10. The user can move the pusher member 440 forward (move to the left direction in the drawing) against the urging force of the urging member 451, by moving the lever portion 453 connected to the urgingmember 451 via the linkmechanism 452 in the direction of the arrow b in the drawing. When the pusher member 440 moves forward, the gas g such as air contained in the syringe main body 431 is sent to the tube 470 via the port portion 432. Further, when the operating force (gripping force) of the lever portion 453 is relaxed, the pusher member 440 is retracted (moved to the right direction in the drawing) by the urging force of the urging member 451.

As described above, when the filling mechanism 420 according to the present embodiment delivers the drug solution from the drug solution filling device 400 to the drug solution reservoir instrument 200, since the direction in which the pusher member 440 is moved against the urging force of the urging member 451 (the direction of the arrow b) coincides with the operation direction (the direction of the arrow b) of the lever portion 453, the user can easily grasp the progress state of the filling operation.

As illustrated in Fig. 10, the filling amount adjustment mechanism 480 has a feed screw portion 481, a movable member 482 connected to the feed screw portion 481, an engaging portion 483 connectable to an engaging piece 245 (see Fig. 3) provided in the liquid sending mechanism 220 of the drug solution reservoir instrument 200, an indication piece 484 indicating the initial filling amount of the drug solution reservoir instrument 200, and a rotary dial 485 for operating the feed screw portion 481.

When the rotary operation is performed by the user, the rotary dial 485 rotates the feed screw portion 481 to move the movable member 482 connected to the feed screw portion 481. Specifically, when the rotary dial 485 is rotated in a direction of an arrow r illustrated in Figs. 10 and 11, the movable member 482 moves in a direction of an arrow c (an upward direction), and when the rotary dial 485 is rotated in a direction of an arrow r' , the movable member 482 moves in a direction of an arrow c' (a downward direction).

When the movable member 482 moves in the vertical direction (c-c' direction) as described above, the engaging portion 483 and the indication piece 484 integrally provided on the movable member 482 also similarly move in the vertical direction.

The engaging portion 483 is connected to the engaging piece 245 provided in the liquid sending mechanism 220 of the drug solution reservoir instrument 200 in a state in which the drug solution filling unit 100 has been mounted to the drug solution filling device 400 (see Fig. 3). When the movable member 482 is moved in the vertical direction as described above, the engaging portion 483 also moves together with the movable member 482. Further, when the engaging portion 483 moves in a state of being connected to the engaging piece 245, the shaft portion 243 of the liquid sending mechanism 220 to which the engaging piece 245 is attached also moves (see Fig. 3). In this manner, the movement of the movable member 482 is converted into the movement of the shaft portion 243 of the liquid sending mechanism 220. Further, the shaft portion 243 of the liquid sending mechanism 220 moves and the gasket 241 moves together with the shaft portion 243, thereby adjusting the volume of the drug solution reservoir portion 230 in which the drug solution can be filled.

As illustrated in Fig. 11, the rotary dial 485 is exposed to the outside via the window portion 414d. Therefore, the user can easily rotate the rotary dial 485 with the fingers. When the rotary dial 485 is rotated in the direction of the arrow r, the indication piece 484 moves in the direction of the arrow c. The user can visually confirm that the volume of the drug solution that can be filled in the drug solution reservoir portion 230 decreases, by movement of the indication piece 484 in the direction of the arrow c. Further, when the rotary dial 485 is rotated in the direction of the arrow r' , the indication piece 484 moves in the direction of the arrow c'. The user can visually confirm that the volume of the drug solution that can be filled in the drug solution reservoir portion 230 increases by the movement of the indication piece 484 in the direction of the arrow c'.

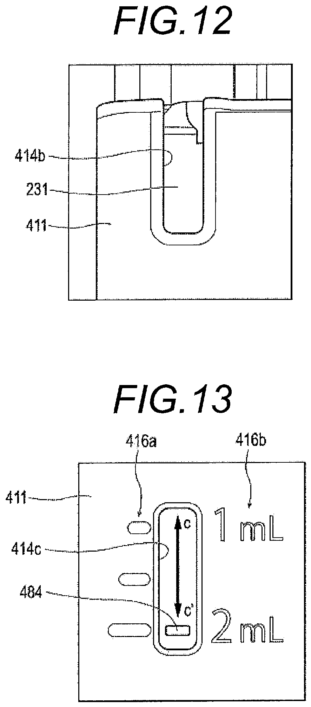

As illustrated in Fig. 13, a scale 416a and a numeral 416b indicating the reading value of the scale 416a are attached to the periphery of the window portion 414c for exposing the indication piece 484. The user can easily grasp the initial filling amount of the drug solution that can be filled in the drug solution reservoir portion 230 (a capacity when starting the administration of the drug solution using the drug solution administration device 600 is started), by confirming the scale 416a and the numeral 416b on the basis of the position of the indication piece 484. Further, in the present embodiment, a relatively large scale 416a or the numeral 416b is attached to the outer surface of the main body portion 411 of the exterior case 410. Therefore, the user can easily grasp the initial filling amount of the drug solution by visual observation.

As illustrated in Fig. 12, the window portion 414b formed in the main body portion 411 of the exterior case 410 makes it possible to visually recognize the reservoir portion main body 231 of the drug solution reservoir instrument 200 from the outside. Since the user can visually confirm the progress of the filling operation of the reservoir portion main body 231 via the window portion 414b, while filling the reservoir portion main body 231 with the drug solution, it is possible to preferably prevent the drug solution more than necessary from being filled in the reservoir portion main body 231.

As illustrated in Fig. 10, the holding mechanism 490 of the drug solution filling device 400 has a predetermined holding member 491, and an urging member 493 that imparts the urging force to the holding member 491.

The holding member 491 presses the drug solution reservoir instrument 200 in a state in which the drug solution filling unit 100 has been mounted to the drug solution filling device 400, thereby preventing the drug solution reservoir instrument 200 from being misaligned or the like.

The urging member 493 can be constituted by, for example, a known coil spring. The urging member 493 imparts an urging force (an elastic force) to the holding member 491 along a direction indicated by an arrow d of Fig. 10. The holding member 491 receives the urging force imparted by the urging member 493, and presses the drug solution reservoir instrument 200 inserted into the exterior case 410.

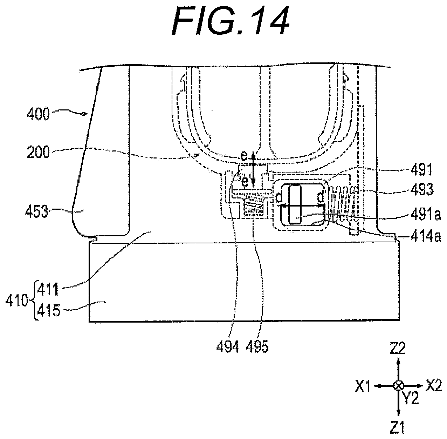

As illustrated in Fig. 14, the holding member 491 has a lever portion 491a exposed from a window portion 414a formed in the main body portion 411 of the exterior case 410.

Further, a push-up member 494 that operates when the drug solution filling unit 100 is detached from the drug solution filling device 400, and a urging member 495 that imparts the urging force to the push-up member 494 are disposed inside the main body portion 411 of the exterior case 410. When the drug solution filling unit 100 is mounted to the drug solution filling device 400, the urging member 495 is pressed in the direction of arrow e' (the downward direction in the drawing) by the drug solution reservoir instrument 200 and contracted. Further, the urging member 495 can be constituted by, for example, a known coil spring.

When the drug solution filling unit 100 is detached from the drug solution filling device 400, the user moves the lever portion 491a exposed from the window portion 414a in the direction of the arrow d' in Fig. 14. When this operation is performed, the pressing of the drug solution reservoir instrument 200 using the urging force of the urging member 493 is released. At the same time when the pressing of the drug solution reservoir instrument 200 using the urging member 493 released, the urging member 495 extends in the direction of arrow e (the upward direction in the drawing), thereby causing the upper portion side of the drug solution filling unit 100 (the upper portion side of the filling adapter 300) to protrude from the exterior case 410 of the drug solution filling device 400. As a result, the user can extract the drug solution filling unit 100 from the exterior case 410 of the drug solution filling device 400.

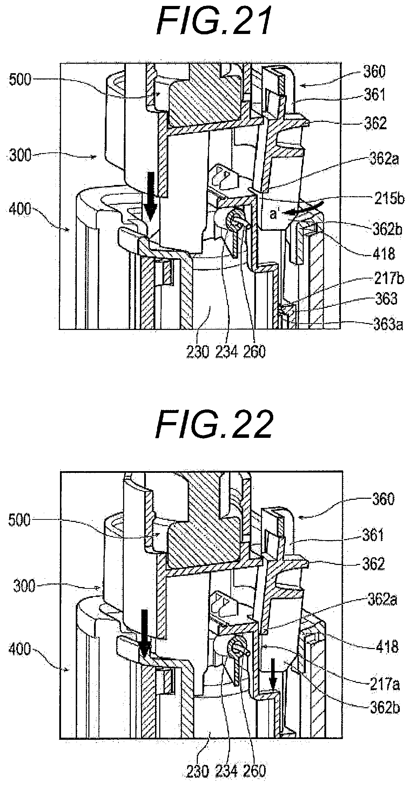

As illustrated in Figs. 18 and 21, the drug solution filling device 400 has a restriction releasing portion 418 which releases the restriction of the lock mechanism 360 with mounting of the drug solution filling unit 100 to the drug solution filling device 400.

The restriction releasing portion 418 is constituted by ribs (convex portions) formed on the exterior case 410 of the drug solution filling device 400. The restriction releasing portion 418 is formed at a position facing the lock mechanism 360 of the filling adapter 300 in the vicinity of the upper opening portion 412 of the exterior case 410. The restriction releasing portion 418 has a shape protruding into the exterior case 410.

The materials of each of the seal members 233, 235, 333, and 353 according to the present embodiment are not particularly limited, but it is possible to adopt elastic materials, such as various rubber materials such as natural rubber, butyl rubber, isoprene rubber, butadiene rubber, styrene-butadiene rubber and silicone rubber, various thermoplastic elastomers such as polyurethane type, polyester type, polyamide type, olefin type and styrene type, or a mixture thereof.

Further, for example, a known needle member or the like in which a flow path is formed inside can be appropriately used for each of the needle tubes 261, 321 and 471 according to the present embodiment, and the material, size, shape, and the like are not particularly limited. Further, tubes (hollow members) made of metal, resin, or the like can be used suitably for each of the tubes 260 and 470 according to the present embodiment.

In addition, the materials of the exterior case 210 and 410 and the adapter main body portion 310 according to the present embodiment are not particularly limited, but it is possible to adopt, for example, a resin material such as polypropylene, polyethylene terephthalate, polymethyl methacrylate, and polycarbonate.

### <Drug solution container>

As illustrated in Figs. 1 and2, the drug solution container 500 includes a main body portion 510 which contains the drug solution to be administered to a patient, a lid portion 520 configured to be connectable to and separable from the filling adapter 300, and a seal member 523 arranged inside the lid portion 520.

The drug solution container 500 is constituted by, for example, a known vial bottle. The drug solution contained in the drug solution container 500 is, for example, insulin. Further, it is preferable that the drug solution container 500 is formed to be transparent or translucent, for example, so that the residual amount of the drug solution therein or the like can be visually recognized from the outside.

As described above, the user who uses the drug solution administration device 600 (see Fig. 25) sets the drug solution container 500 in the drug solution filling unit 100 in use. At this time, the user causes the first needle tube 321 provided in the filling adapter 300 to penetrate through the seal member 523 of the drug solution container 500. The inside of the drug solution container 500 and the drug solution reservoir portion 230 of the drug solution reservoir instrument 200 communicate with each other via the first needle tube 321 (see Fig. 2).

The procedure of using the drug solution filling unit 100 will be briefly described with reference to Fig. 15.

First, when administering the drug solution using the drug solution administration device 600, the user takes out the drug solution filling unit 100 from a predetermined packaging material or the like. The position of the filling adapter 300 is held at a position (the non-penetrating position P1) at which the first needle tube 321 on the filling adapter 300 side does not penetrate the seal member 233 on the drug solution reservoir portion 230 side, in the state of being taken out from the packaging material, and the second needle tube 261 on the drug solution reservoir instrument 200 side is held at a position (non-penetrating position P1) at which the second needle tube 261 does not penetrate the seal member 333 on the filling adapter 300 side.

Next, the user mounts the drug solution container 500 on the drug solution filling unit 100 (see STEP 1 of Fig. 15). Further, the user mounts the drug solution filling unit 100 on the drug solution filling device 400.

When the operation of mounting the drug solution filling unit 100 on the drug solution filling device 400 is performed, the position of the filling adapter 300 moves to a position (the penetrating position P2) at which the first needle tube 321 on the filling adapter 300 side penetrates the seal member 233 on the drug solution reservoir portion 230 side, and the second needle tube 261 on the drug solution reservoir instrument 200 side penetrates the seal member 333 on the filling adapter 300 side. Also, the position of the filling adapter 300 is held at the penetrating position P2. In this state, the user operates the drug solution filling device 400 to deliver the drug solution from the drug solution container 500 to the drug solution reservoir portion 230 (see STEP 2 of Fig. 15).

Further, the penetration of the third needle tube 471 (see Fig. 5) with respect to the seal member 353 disposed in the second connection port portion 350 of the filling adapter 300 is performed when the operation of mounting the drug solution filling unit 100 to the drug solution filling device 400 is performed.

After filling of the drug solution to the drug solution reservoir portion 230 is completed, the user detaches the drug solution filling unit 100 from the drug solution filling device 400 (see STEP 3 of Fig. 15). Thereafter, the drug solution container 500 is detached from the filling adapter 300, and the filling adapter 300 is detached from the drug solution reservoir instrument 200. As illustrated in Fig. 25, the drug solution reservoir instrument 200 filled with the drug solution is connected to the other members (a reuse portion 610 and a cradle 630) constituting the drug solution administration device 600. By performing the above procedure, the user can start administration of the drug solution using the drug solution administration device 600.

A procedure of using the drug solution filling device 400 will be briefly described with reference to Fig. 16.

Fig. 16 (A) illustrates a state in which the drug solution filling unit 100 has been mounted to the drug solution filling device 400 at the time of starting the filling operation.

The main body portion 510 of the drug solution container 500 is connected to the drug solution reservoir portion 230 of the drug solution reservoir instrument 200 via the first needle tube 321. Further, the drug solution reservoir portion 230 is connected to the syringe 430 of the drug solution filling device 400 via a predetermined filling flow path T1.

The filling flow path T1 is constituted by a tube 470 of the drug solution filling device 400, a third needle tube 471 of the drug solution filling device 400, a second connection port portion 350 of the filling adapter 300, a flow passage 341 of the filling adapter 300, a first connection port portion 330 of the filling adapter 300, a second needle tube 261 of the drug solution reservoir instrument 200, and a tube 260 of the drug solution reservoir instrument 200 (see Fig. 2). As described above, the ventilation filter 345 is disposed in the flow passage 341 of the filling adapter 300 (see Fig. 5).

The drug solution container 500 is connected to the filling adapter 300 in a state in which the lid portion 520 has been disposed at the lower side in the vertical direction (see Fig. 1) . Thus, the lid portion 520 side (the lower side of Fig. 16 (A)) of the main body portion 510 of the drug solution container 500 is in the state of being filled with the drug solution M1, and a space capable of holding the gas g such as air is formed on the bottom portion side (the upper side of Fig. 16(A)) of the main body portion 510 of the drug solution container 500.

Next, as illustrated in Fig. 16 (B), the user performs an operation of delivering the drug solution from the drug solution container 500 to the drug solution reservoir portion 230.

The user grips the lever portion 453 of the drug solution filling device 400 and moves the pusher member 440 of the drug solution filling device 400 forward (move in the direction of arrow b of Fig. 10). When the pusher member 440 moves forward, the gas g such as air is pumped to the drug solution container 500 via the filling flow path T1 of the drug solution filling device 400, the drug solution reservoir portion 230, and the first needle tube 321. The drug solution container 500 is pressurized by the gas g such as pumped air. The gas g such as air pumped to the drug solution container 500 moves inside the main body portion 510 of the drug solution container 500 and stays on the bottom portion side of the main body portion 510 (the upper side of Fig. 16(B)).

The user releases the gripping of the lever portion 453 of the drug solution filling device 400 in a state in which the inside of the drug solution container 500 has been pressurized. When the gripping of the lever portion 453 is released, the pusher member 440 of the drug solution filling device 400 moves backward (moves in the direction of the arrow b' of Fig. 10) by the urging force of the urging member 451. When the pusher member 440 moves backward, a negative pressure (suction pressure) acts on the drug solution reservoir portion 230 via the filling flow path T1. As a result, the drug solution M1 in the drug solution container 500 moves to the drug solution reservoir portion 230.

Thereafter, similarly, as illustrated in Fig. 16(B), by repeating the gripping and releasing of gripping of the lever portion 453 of the drug solution filling device 400 to alternately perform the pressurization and depressurization (opening), it is possible to fill the desired amount of drug solution M1 in the drug solution reservoir portion 230.

As illustrated in Fig. 16(C), when the drug solution M1 fills the drug solution reservoir portion 230 and further reaches the ventilation filter 345, the drug solution M1 cannot move (cannot be sucked). At this timing, the filling of the drug solution into the drug solution reservoir portion 230 is completed.

After completing the filling operation of the drug solution M1, the user detaches the drug solution filling unit 100 from the drug solution filling device 400. Next, the user detaches the filling adapter 300 from the drug solution reservoir instrument 200. At this time, the user pulls out the first needle tube 321 of the filling adapter 300 from the seal member 233 of the drug solution reservoir portion 230. The seal member 233 of the drug solution reservoir portion 230 maintains good sealing property and prevents the drug solution M1 from leaking from the first connection port portion 232 of the drug solution reservoir portion 230. Also, the user pulls out the second needle tube 261 on the drug solution reservoir instrument 200 side from the seal member 333 of the filling adapter 300.

By the above procedure, it is possible to fill the drug solution reservoir instrument 200 with a desired amount of the drug solution M1.

Next, the operation of the lock mechanism 360 provided in the filling adapter 300 will be described referring to Figs. 17 to 24.

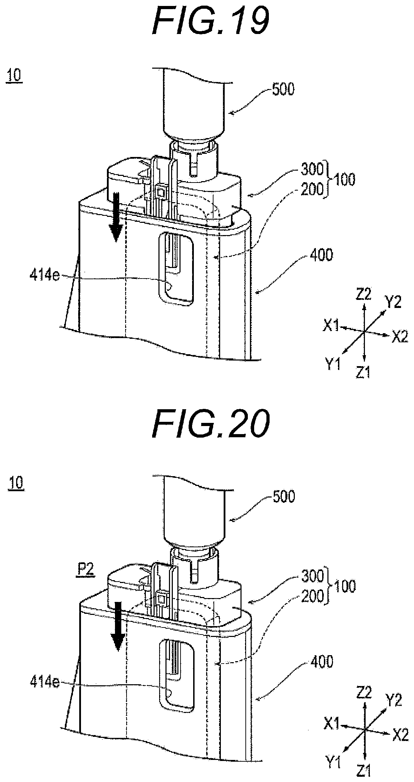

Fig. 17 illustrates a state when the drug solution filling unit 100 is mounted to the drug solution filling device 400. Fig. 18 illustrates a cross-sectional view taken along the arrow 18A-18A illustrated in Fig. 17. Figs. 19 and 20 are perspective views illustrating a procedure for mounting the drug solution filling unit 100 to the drug solution filling device 400 from the state illustrated in Fig. 17. Figs. 21 to 23 are diagrams illustrating a procedure for mounting the drug solution filling unit 100 to the drug solution filling device 400, and correspond to the cross-sectional view illustrated in Fig. 18. Fig. 24 is a cross-sectional view illustrating a main part of the lock mechanism 360.

In the state illustrated in Fig. 17, the filling adapter 300 is disposed at the non-penetrating position P1. In this state, a relative movement of the filling adapter 300 with respect to the drug solution reservoir instrument 200 is restricted by the lock mechanism 360. More specifically, as illustrated in Fig. 18, a portion 362a of the stopper portion 362 of the lock mechanism 360 abuts on the abutting portion 215a of the first lock portion 215, and the claw portion 363 of the lock mechanism 360 is caught by the second groove portion 217b.

As illustrated in Fig. 19, the restriction of the lock mechanism 360 can be released, by inserting the drug solution filling unit 100 into the drug solution filling device 400, from the state illustrated in Fig. 17. As illustrated in Fig. 21, when the filling adapter 300 is pushed into the drug solution filling device 400, the stopper portion 362 of the lock mechanism 360 brings the lower end portion 362b into contact with the restriction releasing portion 418 of the drug solution filling device 400. The restriction releasing portion 418 releases the abutment between the stopper portion 362 and the first lock portion 215 by pushing the stopper portion 362 as indicated by an arrow a' . As a result, a portion 362a of the stopper portion 362 enters the groove portion 215b of the first lock portion 215.

Further, as illustrated in Fig. 21, the claw portion 363 of the lock mechanism 360 comes out of the second groove portion 217b when the stopper portion 362 is pushed in as indicated by the arrow a' (see Fig. 7).

As described above, when the abutment between the stopper portion 362 of the lock mechanism 360 and the abutting portion 215a of the first lock portion 215 is released, and further, when the claw portion 363 of the lock mechanism 360 comes out of the second groove portion 217b, the filling adapter 300 is in a state capable of relatively moving to slide with respect to the drug solution reservoir instrument 200.

When the drug solution filling unit 100 is moved as illustrated in Fig. 20 in a state in which the restriction of the lock mechanism 360 has been released, the filling adapter 300 can move to the penetrating position P2. At this time, as illustrated in Figs. 22 and 23, the stopper portion 362 of the lock mechanism 360 moves along the first groove portion 217a. Further, as illustrated in Fig. 24, the claw portion 363 of the lock mechanism 360 moves to the third groove portion 217c and is caught by the third groove portion 217c (see Fig. 7) . When the filling adapter 300 moves to this position, the filling adapter 300 enters a state in which the movement has been restricted by the claw portion 363. Therefore, the filling adapter 300 is held at the penetrating position P2.

When the drug solution reservoir instrument 200 is detached from the filling adapter 300 after completion of the filling of the drug solution to the drug solution reservoir instrument 200, an operation of pushing the lever portion 361 is performed as indicated by an arrow p in Fig. 7. When this operation is performed, the lock mechanism 360 moves in the direction indicated by the arrow a. As the lock mechanism 360 is movable, since the claw portion 363 comes out of the third groove portion 217c, the filling adapter 300 can be detached from the drug solution reservoir instrument 200.

### <Drug solution administration device>

As illustrated in Fig. 25, the drug solution administration device 600 according to the present embodiment has a reuse portion 610, a drug solution reservoir instrument (disposable portion) 200, and a cradle 630.

The reuse portion 610 has a lid body 611, and a drive portion 613 accommodated inside the lid body 611.

The lid body 611 is configured to be attachable to and detachable from the exterior case 210 of the drug solution reservoir instrument 200. The drive portion 613 has a motor 613a and a gear group 613b. The motor 613a can be constituted by, for example, a known stepping motor. The gear group 613b is rotationally driven by the motor 613a. The gear group 613b is configured to be freely engaged with the gear group 251 included in the drug solution reservoir instrument 200.

A battery box 280 including a battery serving as a power supply for driving the motor 613a is disposed in the drug solution reservoir instrument 200. The battery box 280 may be disposed in the reuse portion 610.

The cradle 630 has a main body portion 631, a catheter port 634, and an administration port 635. A needle portion 635a for administering the drug solution to a patient is disposed in the administration port 635.

The drug solution administration device 600 is configured by mutually connecting the reuse portion 610, the drug solution reservoir instrument 200 with the drug solution filled in the drug solution reservoir portion 230, and the cradle 630. Upon use of the drug solution administration device 600, the cradle 630 ismounted (fixed) to a part of the patient's body (for example, an abdomen or a limb). Further, a predetermined operation command is transmitted by a controller (not illustrated) or the like to operate the motor 613a. When the motor 613a is operated, the gear group 613b of the reuse portion 610 operates, and the gear group 251 of the drug solution reservoir instrument 200 operates in conjunction with the operation of the gear group 613b. Further, as the pusher member 240 of the liquid sending mechanism 220 of the drug solution reservoir instrument 200 starts to move, the drug solution is administered to the patient via the needle portion 635a.

After the administration of the drug solution to the patient is completed, the drug solution reservoir instrument 200 is detached from the reuse portion 610. The drug solution reservoir instrument 200 can be discarded after use, and the reuse portion 610 can be reused when the drug solution is separately administered.

Next, the operational effect of the present embodiment will be described.

As described above, the drug solution filling unit 100 according to the present embodiment includes the drug solution reservoir instrument 200 having the drug solution reservoir portion 230 for storing the drug solution, the filling adapter 300 detachably mounted to the drug solution reservoir instrument 200, and the lock mechanism 360 for restricting the relative movement of the filling adapter 300 with respect to the drug solution reservoir instrument 200. In addition, the drug solution reservoir instrument 200 has the exterior case 210 that accommodates the drug solution reservoir portion 230, the first connection port portion 232 provided in the drug solution reservoir portion 230, and the seal member 233 disposed in the first connection port portion 232. In addition, the filling adapter 300 has the first needle tube 321 connected to the drug solution container 500 containing the drug solution and pierced to the seal member 233, and the adapter main body portion 310 to which the first needle tube 321 is attached. Further, in a state in which the filling adapter 300 has been mounted to the drug solution reservoir instrument 200, the lock mechanism 360 is able to hold the relative position of the adapter main body portion 310 with respect to the exterior case 210 at the non-penetrating position P1 at which the first needle tube 321 does not penetrate the seal member 233, and the penetrating position P2 at which the first needle tube 321 penetrates the seal member 233.

According to the drug solution filling unit 100 configured as described above, the user can prevent the first needle tube 321 of the filling adapter 300 from penetrating the seal member 233 of the drug solution reservoir portion 230 before the filling of the drug solution into the drug solution reservoir instrument 200 is started, in the state in which the filling adapter 300 has been mounted to the drug solution reservoir instrument 200. Further, the user can cause the first needle tube 321 of the filling adapter 300 to penetrate the seal member 233 by a simple operation of changing the relative position of the filling adapter 300 with respect to the drug solution reservoir instrument 200. Therefore, the drug solution filling unit 100 can prevent the sealing property of the seal member 233 of the drug solution reservoir portion 230 from being lowered, and can easily perform the operation of causing the first needle tube 321 to penetrate the seal member 233 of the drug solution reservoir portion 230.

The lock mechanism 360 is provided integrally with the filling adapter 300, releases the restriction in accordance with the operation of causing the filling adapter 300 to relatively slide with respect to the drug solution reservoir instrument 200, and enables the movement from the non-penetrating position P1 to the penetrating position P2. Therefore, the user can cause the first needle tube 321 to penetrate the seal member 233, by a simple operation of moving the filling adapter 300 to slide with respect to the drug solution reservoir instrument 200.

The lock mechanism 360 has the claw portion 363 which restricts the relative movement of the filling adapter 300 with respect to the drug solution reservoir instrument 200, by being caught by the respective groove portions 217b and 217c formed in the exterior case 210 of the drug solution reservoir instrument 200. Therefore, the drug solution filling unit 100 can restrict the movement of the filling adapter 300 with a simple configuration in which the claw portion 363 is caught by the respective groove portions 217b and 217c. Further, the drug solution filling unit 100 can release the restriction of movement of the filling adapter 300 by a simple operation of releasing the catching of the clawportion 363 with respect to the respective groove portions 217b and 217c.

The lock mechanism 360 has a lever portion 361 which releases the catching of the claw portion 363 with respect to the respective groove portions 217b and 217c in accordance with the pressing operation. Therefore, by operating the lever portion 361, the user can easily release the catching of the claw portion 363 with respect to the respective groove portions 217b and 217c.

The filling adapter 300 has the flow passage 341 formed in the adapter main body portion 310 and capable of allowing the gas g such as air to flow, the first connection port portion 330 communicating with the flow passage 341, and the seal member 333 disposed in the first connection port portion 330. Further, the drug solution reservoir instrument 200 has the second connection port portion 234 provided at a position different from the first connection port portion 232 in the drug solution reservoir portion 230, the tube 260 connected to the second connection port portion 234, and the second needle tube 261 attached to the tube 260. Further, the second needle tube 261 does not penetrate the seal member 333 when the relative position of the adapter main body portion 310 with respect to the exterior case 210 is at the non-penetrating position P1, and the second needle tube 261 penetrates the seal member 333 when the relative position of the adapter main body portion 310 with respect to the exterior case 210 is at the penetrating position P2. Therefore, the user can prevent the second needle tube 261 from penetrating the seal member 333 of the filling adapter 300 before filling of the drug solution to the drug solution reservoir instrument 200 is started, and can satisfactorily keep the sealing property of the seal member 333. In addition, the user can allow the second needle tube 261 of the drug solution reservoir instrument 200 to penetrate the seal member 333, by a simple operation of changing the relative position of the filling adapter 300 with respect to the drug solution reservoir instrument 200.

The drug solution filling set 10 according to the present embodiment includes the drug solution filling unit 100, and the drug solution filling device 400 to which the drug solution filling unit 100 is detachably mounted. Further, the drug solution filling device 400 has the restriction releasing portion 418 which releases the restriction of the lock mechanism 360 in accordance with the mounting of the drug solution filling unit 100 to the drug solution filling device 400. Since the user can release the restriction of the lock mechanism 360 in accordance with the operation of mounting the drug solution filling unit 100 to the drug solution filling device 400, it is possible to reduce the operation burden of releasing the lock mechanism 360.

The filling adapter 300 according to this embodiment is configured to be attachable to and detachable from the drug solution reservoir instrument 200 having the drug solution reservoir portion 230 for storing the drug solution. The filling adapter 300 includes the lock mechanism 360 which restricts the relative movement of the filling adapter 300 with respect to the drug solution reservoir instrument 200, the adapter main body portion 310 mounted to the exterior case 210 of the drug solution reservoir instrument 200, and the first needle tube 321 connected to the drug solution container 500 containing the drug solution and pierced to the seal member 233 of the drug solution reservoir portion 230. Further, the lock mechanism 360 is configured to be able to hold the relative position of the adapter main body portion 310 with respect to the exterior case 210 at the non-penetrating position P1 at which the first needle tube 321 does not penetrate the seal member 233, and the penetrating position P2 at which the first needle tube 321 penetrates the seal member 233, in the state in which the filling adapter 300 has been mounted to the drug solution reservoir instrument 200.

According to the filling adapter 300 configured as described above, the user can prevent the first needle tube 321 of the filling adapter 300 from penetrating the seal member 233 of the drug solution reservoir portion 230, before filling of the drug solution to the drug solution reservoir instrument 200 is started, in the state in which the filling adapter 300 has been mounted to the drug solution reservoir instrument 200. Further, the user can allow the first needle tube 321 of the filling adapter 300 to penetrate the seal member 233, by a simple operation of changing the relative position of the filling adapter 300 with respect to the drug solution reservoir instrument 200. Therefore, the drug solution filling unit 100 can prevent the sealing property of the seal member 233 of the drug solution reservoir portion 230 from being lowered, and can easily perform the operation of allowing the first needle tube 321 to penetrate the seal member 233 of the drug solution reservoir portion 230.

Although the drug solution filling unit, the drug solution filling set, and the filling adapter according to the present invention have been described through the embodiments, the present invention is not limited only to the contents described in the description, but can be changed appropriately on the basis of the description of the claims.

For example, the shape of the lock mechanism, the connection (coupling) structure for restricting the movement, a positional relation with other members, and the like are not particularly limited. Although an aspect in which the lock mechanism is provided in the filling adapter has been described as an example, the lock mechanism may be provided in the drug solution reservoir instrument. When changing in this way, the groove which cooperates with the lock mechanism can be provided in the filling adapter. Further, the lock mechanism may be configured to release the restriction by moving the drug solution reservoir instrument with respect to the filling adapter. Further, another member configured as a separate body from the filling adapter or the drug solution reservoir instrument may be used as the lock mechanism to restrict the relative movement of the filling adapter and the drug solution reservoir instrument via the lock mechanism or hold the position of the respective members.

In addition, the filling adapter may be configured to connect the drug solution reservoir portion and the drug solution container via at least the first needle tube, and the second needle tube to be connected to the drug solution filling device may not be provided. In addition, the drug solution may be filled, without using the drug solution filling device, for example, by a user manually operating the liquid sending mechanism (the pusher member) of the drug solution reservoir instrument.

In addition, the non-penetrating position described in the embodiment may be set at a position at which a part of the needle tube is pierced (inserted) into the seal member. However, in order to satisfactorily maintain the sealing property of the seal member, it is preferable to set the position, at which the needle tube is not pierced to the seal member, as the non-penetrating position. In addition, each of the penetrating position and the non-penetrating position is not limited to only one position. For example, a plurality of positions may be set as the penetrating position or a plurality of positions may be set as the non-penetrating position.

In addition, the drug solution filling unit, the drug solution filling set, and the filling adapter are not limited to the contents described in the specification, and the shapes, materials and arrangements of each member, the connection form between the members, and the like can be appropriately changed.

This application is based on Japanese Patent Application No. 2016-226230 filed on November 21, 2016.

### Reference Signs List

- 10: DRUG SOLUTION FILLING SET
- 100: DRUG SOLUTION FILLING UNIT
- 200: DRUG SOLUTION RESERVOIR INSTRUMENT (DISPOSABLE PORTION)
- 210: EXTERIOR CASE
- 215: FIRST LOCK PORTION
- 216: SECOND LOCK PORTION
- 217a: FIRST GROOVE PORTION
- 217b: SECOND GROOVE PORTION (GROOVE PORTION)
- 217c: THIRD GROOVE PORTION (GROOVE PORTION)
- 220: LIQUID SENDING MECHANISM
- 230: DRUG SOLUTION RESERVOIR PORTION
- 231: RESERVOIR PORTION MAIN BODY
- 232: FIRST CONNECTION PORT PORTION (FIRST PORT PORTION)
- 233: SEAL MEMBER (FIRST SEAL MEMBER)
- 234: SECOND CONNECTION PORT PORTION (THIRD PORT PORTION)
- 235: SEAL MEMBER
- 240: PUSHER MEMBER
- 261: SECOND NEEDLE TUBE
- 300: FILLING ADAPTER
- 310: ADAPTER MAIN BODY PORTION
- 313: INSERTION PORT
- 321: FIRST NEEDLE TUBE
- 330: FIRST CONNECTION PORT PORTION (SECOND PORT PORTION)
- 333: SEAL MEMBER (SECOND SEAL MEMBER)
- 340: FLOW PATH PARTITION PORTION
- 341: FLOW PASSAGE
- 345: VENTILATION FILTER
- 350: SECOND CONNECTION PORT PORTION
- 353: SEAL MEMBER
- 360: LOCK MECHANISM
- 361: LEVER PORTION
- 362: STOPPER PORTION
- 363: CLAW PORTION
- 400: DRUG SOLUTION FILLING DEVICE
- 410: EXTERIOR CASE
- 418: RESTRICTION RELEASING PORTION
- 420: FILLING MECHANISM
- 430: SYRINGE
- 431: SYRINGE MAIN BODY
- 432: PORT PORTION
- 440: PUSHER MEMBER
- 450: DRIVE PORTION
- 451: URGING MEMBER
- 452: LINK MECHANISM
- 453: LEVER PORTION
- 460: FILLING MACHINE SIDE CONNECTION PORT PORTION
- 470: TUBE
- 471: THIRD NEEDLE TUBE
- 480: FILLING AMOUNT ADJUSTMENT MECHANISM
- 490: HOLDING MECHANISM
- 500: DRUG SOLUTION CONTAINER
- 523: SEAL MEMBER
- 600: DRUG SOLUTION ADMINISTRATION DEVICE
- 610: REUSE PORTION
- 630: CRADLE
- g: GAS
- M1: DRUG SOLUTION
- T1: FILLING FLOW PATH
- P1: NON-PENETRATING POSITION
- P2: PENETRATING POSITION

## Claims

1. A drug solution filling unit (100) comprising:
a drug solution reservoir instrument (200) having a drug solution reservoir portion (230) which stores a drug solution (M1);
a filling adapter (300) detachably mounted to the drug solution reservoir instrument (200); and
a lock mechanism (360) which restricts a relative movement of the filling adapter (300) with respect to the drug solution reservoir instrument (200);
wherein the drug solution reservoir instrument (200) has
an exterior case (210, 410) which accommodates the drug solution reservoir portion (230),
a first port portion (232) provided in the drug solution reservoir portion (230), and
a first seal member (233) disposed in the first port portion (232);
the filling adapter (300) has
a first needle tube (321) connected to a drug solution container (500) containing the drug solution (M1) and that is configured to pierce to the first seal member (233), and
an adapter main body portion (310) to which the first needle tube (321) is attached,
**characterized in that**
the lock mechanism (360) is able to hold a relative position of the adapter main body portion (310) with respect to the exterior case (210, 410), at a non-penetrating position (P1) at which the first needle tube (321) does not penetrate the first seal member (233), and a penetrating position (P2) at which the first needle tube (321) penetrates the first seal member (233), in a state in which the filling adapter (300) has been mounted to the drug solution reservoir instrument (200);
the lock mechanism (360) is provided integrally with the filling adapter (300), releases the restriction with an operation of moving the filling adapter (300) to relatively slide with respect to the drug solution reservoir instrument (200), and enables movement from the non-penetrating position (P1) to the penetrating position (P2);
the lock mechanism (360) has a claw portion (363) which restricts the relative movement of the filling adapter (300) with respect to the drug solution reservoir instrument (200) by being caught by a groove portion (217b, 217c) formed in the exterior case (210, 410): and
the lock mechanism (360) has a lever portion (361, 453) which releases the catching of the claw portion (363) with respect to the groove portion (217b, 217c) with a pressing operation.

2. The drug solution filling unit (100) according to claim 1 , wherein the filling adapter (300) has
a flow passage (341) formed in the adapter main body portion (310) and allowing a gas (g) to flow,
a second port portion (330) communicating with the flow passage (341), and
a second seal member (333) disposed at the second port portion (330), the drug solution reservoir instrument (200) has
a third port portion (234) provided at a position different from the first port portion (232) in the drug solution reservoir portion (230),
a second needle tube (261) attached to the tube (470), and
the second needle tube (261) does not penetrate the second seal member (333) when the relative position of the adapter main body portion (310) with respect to the exterior case (210, 410) is at the non-penetrating position (P1), and the second needle tube (261) penetrates the second seal member (333) when the relative position of the adapter main body portion (310) with respect to the exterior case (210, 410) is at the penetrating position (P2).

3. A drug solution filling set (10) comprising:
the drug solution filling unit (100) according to any one of claims 1 to 2; and
a drug solution filling device (400) to which the drug solution filling unit (100) is detachably mounted,
wherein the drug solution filling device (400) has a restriction releasing portion (418) which releases the restriction of the lock mechanism (360) with mounting of the drug solution filling unit (100) to the drug solution filling device (400).

## Patentansprüche

1. Befüllungseinheit (100) für eine Arzneimittellösung, umfassend:
einen Vorratsbehälter-Apparat (200) für eine Arzneimittellösung, der einen Vorratsbehälterabschnitt (230) für eine Arzneimittellösung aufweist, welcher eine Arzneimittellösung (Ml) speichert;
einen Befüllungsadapter (300), der abnehmbar an dem Vorratsbehälter-Apparat (200) für eine Arzneimittellösung angebracht ist; und
einen Verriegelungsmechanismus (360), der eine relative Bewegung des Befüllungsadapters (300) in Bezug auf den Vorratsbehälter-Apparat (200) für eine Arzneimittellösung einschränkt;
wobei der Vorratsbehälter-Apparat (200) für eine Arzneimittellösung aufweist
ein äußeres Gehäuse (210, 410), das den Vorratsbehälterabschnitt (230) für eine Arzneimittellösung aufnimmt,
einen ersten Anschlussabschnitt (232), der in dem Vorratsbehälterabschnitt (230) für eine Arzneimittellösung vorgesehen ist, und
ein erstes Dichtungselement (233), das in dem ersten Anschlussabschnitt (232) angeordnet ist;
wobei der Befüllungsadapter (300) aufweist
ein erstes Nadelrohr (321), das mit einem Arzneimittellösungsbehälter (500) verbunden ist, der die Arzneimittellösung (Ml) enthält, und das so konfiguriert ist, dass es das erste Dichtungselement (233) durchsticht, und
einen Adapter-Hauptkörperabschnitt (310), an dem das erste Nadelrohr (321) befestigt ist,
**dadurch gekennzeichnet, dass**
der Verriegelungsmechanismus (360) in der Lage ist, eine relative Position des Adapter-Hauptkörperabschnitts (310) in Bezug auf das äußere Gehäuse (210, 410) in einer nicht-durchstechenden Position (P1), in welcher das erste Nadelrohr (321) das erste Dichtungselement (233) nicht durchsticht, und in einer durchstechenden Position (P2), in welcher das erste Nadelrohr (321) das erste Dichtungselement (233) durchsticht, in einem Zustand zu halten, in dem der Befüllungsadapter (300) an dem Vorratsbehälter-Apparat (200) für eine Arzneimittellösung angebracht worden ist;
der Verriegelungsmechanismus (360) einstückig mit dem Befüllungsadapter (300) vorgesehen ist, die Beschränkung bei einem Vorgang des Bewegens des Befüllungsadapters (300) zum relativen Gleiten in Bezug auf den Vorratsbehälter-Apparat (200) für eine Arzneimittellösung aufhebt und eine Bewegung von der nicht-durchstechenden Position (P1) zu der durchstechenden Position (P2) ermöglicht;
der Verriegelungsmechanismus (360) einen Klauenabschnitt (363) aufweist, der die relative Bewegung des Befüllungsadapters (300) in Bezug auf den Vorratsbehälter-Apparat (200) für eine Arzneimittellösung einschränkt, indem er von einem Rillenabschnitt (217b, 217c), der in dem äußeren Gehäuse (210, 410) ausgebildet ist, erfasst wird; und
der Verriegelungsmechanismus (360) einen Hebelabschnitt (361, 453) aufweist, der die Verriegelung des Klauenabschnitts (363) in Bezug auf den Rillenabschnitt (217b, 217c) durch eine Druckbetätigung löst.

2. Befüllungseinheit (100) für eine Arzneimittellösung nach Anspruch 1, wobei der Befüllungsadapter (300) aufweist
einen Strömungsdurchgang (341), der in dem Hauptkörperabschnitt (310) des Adapters ausgebildet ist und das Durchströmen eines Gases (g) ermöglicht,
einen zweiten Anschlussabschnitt (330), der mit dem Strömungsdurchgang (341) in Verbindung steht, und
ein zweites Dichtungselement (333), das an dem zweiten Anschlussabschnitt (330) angeordnet ist, wobei der Vorratsbehälter-Apparat (200) für eine Arzneimittellösung aufweist
einen dritten Anschlussabschnitt (234), der an einer anderen Position als der erste Anschlussabschnitt (232) in dem Vorratsbehälterabschnitt (230) für eine Arzneimittellösung vorgesehen ist,
ein zweites Nadelrohr (261), das an dem Rohr (470) befestigt ist, und
das zweite Nadelrohr (261) nicht in das zweite Dichtungselement (333) eindringt, wenn die relative Position des Adapter-Hauptkörperabschnitts (310) in Bezug auf das Außengehäuse (210, 410) in der nicht eindringenden Position (P1) ist, und das zweite Nadelrohr (261) in das zweite Dichtungselement (333) eindringt, wenn die relative Position des Adapter-Hauptkörperabschnitts (310) in Bezug auf das Außengehäuse (210, 410) in der eindringenden Position (P2) ist.

3. Befüllungsset (10) für eine Arzneimittellösung, umfassend:
die Befüllungseinheit (100) für eine Arzneimittellösung nach einem der Ansprüche 1 bis 2; und
eine Befüllungsvorrichtung (400) für eine Arzneimittellösung, an der die Befüllungseinheit (100) für eine Arzneimittellösung abnehmbar angebracht ist,
wobei die Befüllungsvorrichtung (400) für eine Arzneimittellösung einen Beschränkungsfreigabeabschnitt (418) aufweist, der die Beschränkung des Verriegelungsmechanismus (360) durch die Anbringung der Befüllungseinheit (100) für eine Arzneimittellösung an der Befüllungsvorrichtung (400) für eine Arzneimittellösung freigibt.

## Revendications

1. Unité de versement de solution médicamenteuse (100) comprenant :
un instrument de réservoir de solution médicamenteuse (200) présentant une partie de réservoir de solution médicamenteuse (230) qui stocke une solution médicamenteuse (M1) ;
un adaptateur de versement (300) monté de manière détachable sur l'instrument de réservoir de solution médicamenteuse (200) ; et
un mécanisme de verrouillage (360) qui limite un déplacement relatif de l'adaptateur de versement (300) par rapport à l'instrument de réservoir de solution médicamenteuse (200) ;
dans laquelle l'instrument de réservoir de solution médicamenteuse (200) présente
un boîtier extérieur (210, 410) qui loge la partie de réservoir de solution médicamenteuse (230),
une première partie d'orifice (232) prévue dans la partie de réservoir de solution médicamenteuse (230), et
un premier élément d'étanchéité (233) disposé dans la première partie d'orifice (232) ;
l'adaptateur de versement (300) présente
un premier tube d'aiguille (321) relié à un contenant de solution médicamenteuse (500) contenant la solution médicamenteuse (M1) et qui est configuré pour percer le premier élément d'étanchéité (233), et
une partie de corps principal d'adaptateur (310) à laquelle le premier tube d'aiguille (321) est fixé,
**caractérisé en ce que**
le mécanisme de verrouillage (360) est capable de maintenir une position relative de la partie de corps principal d'adaptateur (310) par rapport au boîtier extérieur (210, 410), au niveau d'une position de non-pénétration (P1) au niveau de laquelle le premier tube d'aiguille (321) ne pénètre pas le premier élément d'étanchéité (233), et une position de pénétration (P2) au niveau de laquelle le premier tube d'aiguille (321) pénètre le premier élément d'étanchéité (233), dans un état dans lequel l'adaptateur de versement (300) a été monté sur l'instrument de réservoir de solution médicamenteuse (200) ;
le mécanisme de verrouillage (360) est prévu d'une seule pièce avec l'adaptateur de versement (300), libère la limitation avec un actionnement de déplacement de l'adaptateur de versement (300) pour qu'il glisse de manière relative par rapport à l'instrument de réservoir de solution médicamenteuse (200), et permet un déplacement à partir de la position de non-pénétration (P1) vers la position de pénétration (P2) ;
le mécanisme de verrouillage (360) présente une partie de griffe (363) qui limite le déplacement relatif de l'adaptateur de versement (300) par rapport à l'instrument de réservoir de solution médicamenteuse (200) en étant attrapé par une partie de rainure (217b, 217c) formée dans le boîtier extérieur (210, 410) ; et
le mécanisme de verrouillage (360) présente une partie de levier (361, 453) qui libère l'attrapage de la partie de griffe (363) par rapport à la partie de rainure (217b, 217c) avec une opération de pressage.

2. Unité de versement de solution médicamenteuse (100) selon la revendication 1, dans laquelle l'adaptateur de versement (300) présente
un passage d'écoulement (341) formé dans la partie de corps principal d'adaptateur (310) et permettant à un gaz (g) de s'écouler,
une deuxième partie d'orifice (330) communiquant avec le passage d'écoulement (341), et
un deuxième élément d'étanchéité (333) disposé au niveau de la deuxième partie d'orifice (330), l'instrument de réservoir de solution médicamenteuse (200) présente
une troisième partie d'orifice (234) prévue au niveau d'une position différente de la première partie d'orifice (232) dans la partie de réservoir de solution médicamenteuse (230),
un deuxième tube d'aiguille (261) fixé au tube (470), et
le deuxième tube d'aiguille (261) ne pénètre pas le deuxième élément d'étanchéité (333) lorsque la position relative de la partie de corps principal d'adaptateur (310) par rapport au boîtier extérieur (210, 410) est au niveau de la position de non-pénétration (P1), et le deuxième tube d'aiguille (261) pénètre le deuxième élément d'étanchéité (333) lorsque la position relative de la partie de corps principal d'adaptateur (310) par rapport au boîtier extérieur (210, 410) est au niveau de la position de pénétration (P2).

3. Ensemble de versement de solution médicamenteuse (10) comprenant :
l'unité de versement de solution médicamenteuse (100) selon l'une quelconque des revendications 1 à 2 ; et
un dispositif de versement de solution médicamenteuse (400) sur lequel l'unité de versement de solution médicamenteuse (100) est montée de manière détachable,
dans lequel le dispositif de versement de solution médicamenteuse (400) présente une partie de libération de limitation (418) qui libère la limitation du mécanisme de verrouillage (360) avec le montage de l'unité de versement de solution médicamenteuse (100) sur le dispositif de versement de solution médicamenteuse (400).
